# EUROPÄISCHE PATENTSCHRIFT

(11) **EP 1 017 668 B1**
(45) Veröffentlichungstag und Bekanntmachung des Hinweises auf die Patenterteilung: **19.03.2003**
(21) Anmeldenummer: 98950020.2
(22) Anmeldetag: 10.09.1998
(51) Int. Cl.: C07C 237/10, C07C 237/22, A01N 37/20, C08L 83/04, C08L 83/06

(54) **VERFAHREN ZUR HERSTELLUNG VON GLUCOPROTAMINEN**
METHOD FOR PRODUCING GLUCOPROTAMINES
PROCEDE POUR LA PREPARATION DE GLUCOPROTAMINES

(30) Priorität: 19.09.1997 DE 19741356
(43) Veröffentlichungstag der Anmeldung: 12.07.2000
(73) Patentinhaber: Cognis Deutschland GmbH & Co. KG, 40589 Düsseldorf (DE)
(72) Erfinder: BOHLANDER, Ralf, D-40599 Düsseldorf (DE); JÄNSCH, Sven, D-40591 Düsseldorf (DE); SPRINGER, Dirk, D-42781 Haan (DE)
(86) Internationale Anmeldenummer: EP9805774
(87) Internationale Veröffentlichungsnummer: WO99015496

(56) Entgegenhaltungen:
- EP-A- 0 156 275
- DE-A- 3 930 410
- US-A- 4 584 125

## Beschreibung

Die Erfindung betrifft ein Verfahren zur Herstellung von antimikrobiellen Wirkstoffen vom Glucoprotamintyp unter Mitverwendung von Aminosiliconen sowie deren Verwendung als Antischaummittel.

### Stand der Technik

Aus der Deutschen Offenlegungsschrift **DE-A1 3410956** (Henkel) sind antimikrobiell wirksame Substanzen bekannt, die man durch Umsetzung von N-substituierten Propylendiaminen mit 2-Aminoglutarsäureestern erhält. Die bei dieser Reaktion gebildeten Amide, die unter der Bezeichnung Glucoprotamin® im Handel sind, können gegebenenfalls mit Alkylenoxiden und/oder Säuren derivatisiert werden. Eine Übersicht zu den antimikrobiellen Eigenschaften dieser Stoffe ist in **Hyg.Med. 17, 529 (1992)** erschienen. Aus der **DE-A1 4340124** (Ecolab) ist ferner die Verwendung von Glucoprotamin® als viruzider Wirkstoff bekannt.

Die bekannten Aminoglutarsäureesteramide weisen jedoch den Nachteil auf, daß sich Konzentrate - also ihre wäßrige Zubereitungen mit einem Aktivsubstanzgehalt im Bereich von etwa 20 bis 50 Gew.-% - häufig weder als farb- noch als lagerstabil erweisen. In der Praxis zeigen diese Mittel vielmehr die Tendenz, im Laufe der Lagerung einzudicken, Kristalle auszuscheiden und einzutrüben.

Die komplexe Aufgabe der Erfindung hat folglich darin bestanden, ein möglichst einfaches Verfahren zur Verfügung zu stellen, mit dessen Hilfe sich in hoher Ausbeute und Selektivität Glucoprotamine mit ausgezeichneter Farbqualität herstellen lassen, die in wäßriger Verdünnung über eine niedrige Viskosität und eine verbesserte Lagerstabilität verfügen.

### Beschreibung der Erfindung

Gegenstand der Erfindung ist ein Verfahren zur Herstellung von Glucoprotaminen durch Kondensation von N-substituierten Propylendiaminen mit 2-Aminoglutarverbindungen, bei dem man die Reaktion in Gegenwart von Aminosiliconen durchführt.

Überraschenderweise wurde gefunden, daß die Mitverwendung von Aminosiliconen nicht nur eine Verminderung der Schaumentwicklung während der Kondensation bewirkt, was aus verfahrenstechnischen Gründen erwünscht ist, sondern auch zu einer Steigerung der Raum/Zeit-Ausbeute führt. Durch die Schauminhibierung läßt sich die Wasserabspaltung sehr genau kontrollieren, so daß der gewünschte Kondensationsgrad exakt eingestellt werden kann.

Nicht zu erwarten war ferner, daß durch diese Maßnahme gleichzeitig auch Produkte erhalten werden, die sich gegenüber dem Stand der Technik durch eine wesentlich hellere Farbe und deutlich verbesserte Lagerbeständigkeit auszeichnen.

### N-substituierte Propylendiamine

Die als Ausgangsstoffe einzusetzenden N-substituierten Propylendiamine folgen vorzugsweise der Formel (I),

**R**^{**1**}**-NH-CH**_{**2**}**CH**_{**2**}**CH**_{**2**}**NH**_{**2**} (I)

in der R¹ für einen linearen Alkylrest mit 6 bis 22 und insbesondere 12 bis 14 Kohlenstoffatomen steht. Derartige Stoffe können nach den gängigen Methoden der organischen Synthese erhalten werden, beispielsweise durch Umsetzung der entsprechenden Alkylamine mit Acrylnitril und nachfolgende Hydrierung [vgl. z.B. **FR-B 1351793].** Aus anwendungstechnischen Gründen haben sich N-substituierte Propylendiamine bewährt, bei denen im Alkylrest 12 bis 14 Kohlenstoffatome enthalten sind, wobei der C₁₂-Anteil vorzugsweise 65 bis 70 Mol-% ausmacht.

### 2-Aminoglutarderivate

Die als zweite Einsatzkomponente in Frage kommenden 2-Aminoglutarderivate folgen vorzugsweise der Formel **(II)**, 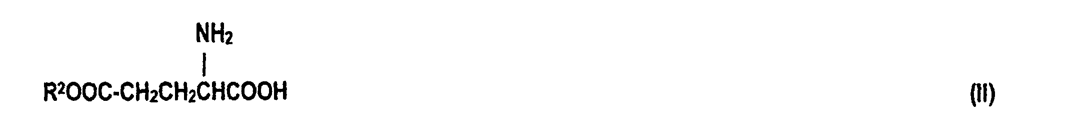 in der R² für Wasserstoff oder einen Alkylrest mit 1 bis 4 Kohlenstoffatomen steht. Verfahren zu ihrer Herstellung sind beispielsweise den Druckschriften **DE-AS 2158562, DE-OS 1493991** und **DE-AS 1254635** zu entnehmen. Üblicherweise wird D- oder L-Glutaminsäure oder deren Racemate eingesetzt, wobei der Einsatz der L-Form bevorzugt ist. Anstelle der Säure können auch deren Methyl-, Ethyl-, Propyl- oder Butylester verwendet werden. Geht man von freier Glutaminsäure aus, so kann man die Kondensation ohne Lösemittel durchführen und das bei der Reaktion entstehende Wasser direkt abdestillieren.

### Kondensationsreaktion

Die Kondensation wird in an sich bekannter Weise durchgeführt, d.h. die N-substituierten Propylendiamine und die 2-Aminoglutarderivate werden üblicherweise im Molverhältnis 1 : 1 bis 1 : 2 eingesetzt. In der Regel findet die Kondensation bei Temperaturen im Bereich von 60 bis 175°, vorzugsweise 100 bis 150°C, statt.

### Aminosilicone

Bei den erfindungsgemäß einzusetzenden Aminosiliconen handelt es sich um im Handel befindliche Stoffe. Vorzugsweise werden solche Polymere eingesetzt, die 50 bis 2.000, vorzugsweise 100 bis 1.000 und insbesondere 200 bis 800, Monomereinheiten der Formeln (**IIIa**) und (**IIIb**) enthalten, 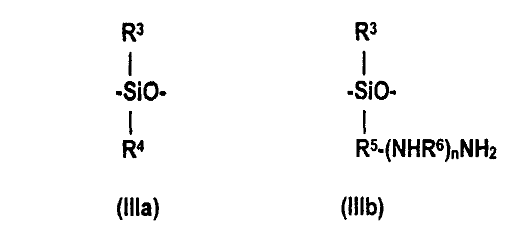 in der R³ und R⁴ unabhängig voneinander für Alkylgruppen mit 1 bis 8 Kohlenstoffatomen, R⁵ und R⁶ unabhängig voneinander für gegebenenfalls hydroxysubstituierte Alkylengruppen mit 1 bis 8 Kohlenstoffatomen und n für 0 oder Zahlen von 1 bis 3 steht. Vorzugsweise werden solche Aminosilicone der Formel (**III**) eingesetzt, in der R³ und R⁴ für Methylgruppen stehen. Besonders bevorzugt werden solche Aminosilicone der Formel (III), die einen Stickstoffgehalt von 0,1 bis 5, insbesondere von 0,5 bis 2, Gew.-% aufweisen. Zur Herstellung dieser Siliconverbindungen können beispielsweise Organopolysiloxane und aminofunktionelle Silane bzw. Siloxane äquilibriert werden, wie dies in der Patentschrift **US 4,584,125** beschrieben wird. Als Organo-polysiloxane eignen sich beispielsweise Hexamethylcyclotrisiloxan, Octamethylcyclotrisiloxan, mit Trimethylsiloxy-Endgruppen verschlossene Dimethyl- bzw. Diethylpolysiloxane. Beispiele für geeignete aminofunktionelle Siloxane sind γ-Aminopropyltrimethoxysilan, γ-Aminopropyltriethoxysilan und N-(β-Aminoethyl)-γ-aminopropyltrimethoxysilan. Die Aminosilicone werden üblicherweise in Mengen von 0,0001 bis 0,2, vorzugsweise 0,001 bis 0,1 und insbesondere 0,01 bis 0,05 Gew.-% - berechnet auf das Glucoprotamin - eingesetzt. Ein weiterer Vorteil des Verfahrens besteht ferner darin, daß die Ver-wendung organischer Schleppmittel zum Wasseraustrag, wie in der Druckschrift **EP-B1 0156275** beschrieben, nicht erforderlich ist, was den technischen Aufwand bei der Herstellung deutlich ver-ringert.

### Gewerbliche Anwendbarkeit

Die nach dem erfindungsgemäßen Verfahren erhältlichen Glucoprotamine sind im Vergleich mit den Produkten des Stands der Technik erheblich hellfarbiger, so daß sich erstmals auch farblose Anwendungslösungen herstellen lassen. Entgegen den bisher bekannten Verfahren lassen sich bei Mitverwendung von Aminosiliconen während der Herstellung auch höher konzentrierte Wirkstofflösungen erhalten, die wegen des Fehlens unerwünschter Polymere, welche leicht ausflocken können, eine deutlich verbesserte Lagerstabilität und eine niedrigere Viskosität aufweisen. Ein weiterer Gegenstand der Erfindung betrifft daher die Verwendung von Aminosiliconen als Entschäumer und Stabilisatoren in der Kondensation von N-substituierten Propylendiaminen mit 2-Aminoglutarderivaten.

### Beispiele

**Vergleichsbeispiel V1.** Im Verlauf von 6 h wurden unter Überleiten von Stickstoff 501,8 g (2 Mol) eines Gemisches aus 70 Mol-% Dodecyl- und 30 Mol-% Tetradecylpropylendiamin und 322,2 g (2 Mol) L-Glutaminsäure-5-methylester unter Rühren auf maximal 133°C (Sumpf) erhitzt, wobei das bei 95 bis 100°C (Sumpf) entstandene Methanol abdestilliert wurde. Nach dem Ende der Reaktion wurden Methanolreste durch kurzzeitiges Anlegen eines schwachen Vakuums (150 bis 50 mbar) bei gleichzeitigem Rühren aus dem Reaktionsgemisch entfernt. Als Rückstand verblieben 735,8 g (97 % der Theorie) Umsetzungsprodukt aus Alkylpropylendiamin und L-Glutaminester im Molverhältnis 1 : 1 in Form einer beigen Paste mit einem Schmelzpunkt im Bereich von 80 bis 90°C.

**Beispiel 1.** Vergleichsbeispiel V1 wurde unter Mitverwendung von 20 ppm eines Aminosilicons (Magnasoft Ultra® FZ 3710, Union Carbide) wiederholt. Als Rückstand verblieben 743 g (98 % der Theorie) des Umsetzungsproduktes in Form einer hellgelben Paste mit einem Schmelzpunkt im Bereich von 75 bis 80°C.

**Vergleichsbeispiel V2.** Unter Überleiten von Stickstoff als Inertgas wurden in einem Kolben mit angeschlossener Destillierbrücke 125,9 g (0,5 Mol) Dodecyl/tetradecylpropylendiamin und 73,6 (0,5 Mol) L-Glutaminsäure im Verlauf von 5 h auf 175°C (Sumpf) erhitzt. Bei einer Sumpftemperatur von 135 bis 145°C wurde die Hauptmenge des bei der Amidbildung zu erwartenden Wassers unter starker Schaumentwicklung abdestilliert. Als Rückstand verblieben 182 g (95,8 % der Theorie) Umsetzungsprodukt aus Alkylpropylendiamin und L-Glutaminester im Molverhältnis 1 : 1 in Form einer beigen Paste mit einem Schmelzpunkt im Bereich von 80 bis 90°C.

**Beispiel 2.** Vergleichsbeispiel V2 wurde unter Mitverwendung von 10 ppm des Aminosilicons aus Beispiel 1 wiederholt. Die Wasserabspaltung begann bereits bei 125°C ohne Schaumentwicklung. 9 ml Wasser wurden als Destillat bereits nach 4 h und einer Endtemperatur von 150°C erhalten. Als Rückstand verblieben 187 g (98 der Theorie) Umsetzungsprodukt als weiße Paste.

**Vergleichsbeispiel V3.** In einem 250 ml-Kolben wurde unter Überleiten von Stickstoff ein Gemisch aus 25,1 g (0,1 Mol) Dodecyl/tetradecylpropylendiamin, 14,7 g (0,1 Mol) L-Glutaminsäure und 100 ml i-Amylalkohol als Löse- und Schleppmittel unter Rühren am Wasserabscheider etwa 2 h lang unter Rückfluß erhitzt, bis sich nahezu die berechnete Menge Wasser abgeschieden hatte. Die Sumpftemperatur betrug dabei maximal 146°C. Danach wurde die Hauptmenge des i-Amylalkohols im Wasserstrahlvakuum, der Rest im Ölpumpenvakuum abdestilliert (je 1 h). Als Rückstand verblieben 36,1 g (95 % der Theorie) Umsetzungsprodukt in Form einer gelblichen Paste mit einem Schmelzpunkt im Bereich von 72 bis 82°C. Die Volumen/Zeitausbeute betrug 36,1 g/250 ml *4 h = 0,036.

**Beispiel 3.** In einem 250-ml-Kolben wurde ein Gemisch aus 75,3 g (0,3 Mol) Dodecyl/tetradecylpropylendiamin, 44 g (0,3 Mol) L-Glutaminsäure und 5 mg Aminosilicon aus Beispiel 1 ohne Schleppmittel unter Rühren in 3 h von 120 auf 150°C erhitzt, wobei die berechnete Menge Wasser abdestillierte. Als Rückstand verblieben 113 g (96 % der Theorie) Umsetzungsprodukt als hellgelbe Paste mit einem Schmelzpunkt im Bereich von 68 bis 75°C. Die Volumen/Zeitausbeute betrug 113 g/250 ml * 3h = 0,15 und war damit erheblich höher als im Vergleichsbeispiel V3.

**Vergleichsbeispiel V4.** In einem 1-1-Glaskolben mit Kühler wurden 124 g Butyldiglycol und 108 g Dodecyl/tetradecylpropylendiamin unter Stickstoff auf 90°C erhitzt. 122 g L-Glutaminsäure wurden eingerührt und die Mischung innerhalb von 2 h auf 145°C erhitzt. Bei 125°C begann der Reaktionsansatz mit einsetzender Wasserabscheidung stark aufzuschäumen. Der Reaktionsfortgang wurde per Leitfähigkeitsmessung kontrolliert und die Reaktion bei Erreichen von 2,2 mS/cm (5 Gew.-%ige Lösung in Wasser, 20°C) durch schnelles Abkühlen abgebrochen. Es fielen 18 g Destillat an. Bei 80°C wurden weitere 175 g Wasser zugegeben, um eine 50 Gew.-%ige Lösung an Wirksubstanz zu erhalten. Gemäß HPLC-Analyse enthielt das Produkt 19,5 Gew.-% Butyldiglycol und 48,8 g Aktivsubstanz. Die Viskosität nach Höppler betrug bei 20°C 800 mPas. Das Produkt vergelte innerhalb von 3 Tagen, mußte bei 60°C aufgeschmolzen werden und zeigte dann eine Viskosität von 900 mPas.

**Beispiel 4,** Vergleichsbeispiel V4 wurde wiederholt, jedoch schon zu Beginn der Reaktion 15 ppm des Aminosilicons aus Beispiel 1 zugegeben. Die Wasserabspaltung erfolgte ohne Aufschäumen. Es fielen nur 15,8 g Destillat an. Gemäß HPLC-Analyse enthielt das Produkt 20,2 Gew.-% Butyldiglycol und 49,7 g Aktivsubstanz. Die Viskosität nach Höppler betrug bei 20°C 300 mPas. Nach drei Tagen zeigte das Produkt eine praktisch konstante Viskosität und erwies sich auch weiterhin als fließ- und pumpfähig.

**Beispiel 5.** Zum Farbvergleich wurden Desinfektionslösungen folgender Zusammensetzung hergestellt:
20 Gew.-% Wirkstoff gemäß Beispiel 1 bis 4, Vergleichsbeispiel V1 bis V4,
10 Gew.-% Nonylphenol+10EO,
40 Gew.-% Ethanol und
30 Gew.-% Wasser.

Die Testlösungen wurden in einem Lange Photometer LICO 100 (Hazen-Farbskala) untersucht. Eine Referenzlösung, bei der die Wirkstoffe gewichtsgleich gegen Glyoxal ausgetauscht wurden, zeigte eine APHA-Farbe von 5. Die Ergebnisse sind in Tabelle 1 zusammengefaßt:

**Tabelle 1**

| **Farbuntersuchungen** | | | | | | | | |
|---|---|---|---|---|---|---|---|---|
| **Bsp.** | **1** | **2** | **3** | **4** | **V1** | **V2** | **V3** | **V4** |
| APHA | 30 | 40 | 20 | 5 | 120 | 100 | 140 | 50 |

**Beispiel 6.** Die Wirkstoffkonzentrate aus den Beispielen 1 bis 4 und den Vergleichsbeispielen V1 bis V4 wurden in einer Testrezeptur bestehend aus
25 Gew.-% Wirkstoff
60 Gew.-% entmineralisiertes Wasser
10 Gew.-% Phenoxyethanol und
5 Gew.-% Kokosfettalkohol+10-butylether
einem Lagertest unterzogen. Die Ergebnisse sind in Tabelle 2 zusammengefaßt:

**Tabelle 2**

| **Lagerversuche** | | | |
|---|---|---|---|
| **Beispiele** | **Lagerzeit** | | |
| | **14 Tage** | **1 Monat** | **3 Monate** |
| V1 | klar | trübe | ausgeflockt |
| 1 | klar | klar | klar |
| V2 | klar | stark trübe | ausgeflockt |
| 2 | klar | klar | klar |
| V3 | klar | klar | leicht trübe |
| 3 | klar | klar | klar |
| V4 | klar | klar | stark trübe |
| 4 | klar | klar | klar |

## Patentansprüche

1. Verfahren zur Herstellung von Glucoprotaminen durch Kondensation von N-substituierten Propylendiaminen mit 2-Aminoglutarderivate, **dadurch gekennzeichnet, daß** man die Reaktion in Gegenwart von Aminosiliconen durchführt.

2. Verfahren nach Anspruch 1, **dadurch gekennzeichnet, daß** man N-substituierte Propylendiamine der Formel (**I**) einsetzt,
**R**^{**1**}**-NH-CH**_{**2**}**CH**_{**2**}**CH**_{**2**}**NH**_{**2**} (I)
in der R¹ für einen linearen Alkylrest mit 6 bis 22 Kohlenstoffatomen steht.

3. Verfahren nach den Ansprüchen 1 und 2, **dadurch gekennzeichnet, daß** man 2-Aminoglutarderivate der Formel (**II**) einsetzt, 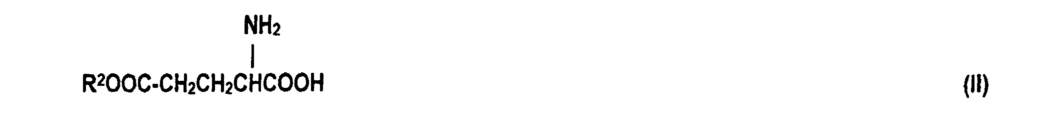 in der R² für Wasserstoff oder einen Alkylrest mit 1 bis 4 Kohlenstoffatomen steht.

4. Verfahren nach den Ansprüchen 1 bis 3, **dadurch gekennzeichnet, daß** man die N-substituierten Propylendiamine und die 2-Aminoglutarderivate im Molverhältnis 1 : 1 bis 1 : 2 einsetzt.

5. Verfahren nach den Ansprüchen 1 bis 4, **dadurch gekennzeichnet, daß** man die Kondensation bei Temperaturen im Bereich von 60 bis 175°C durchführt.

6. Verfahren nach den Ansprüchen 1 bis 5, **dadurch gekennzeichnet, daß** man Aminosilicone einsetzt, die 50 bis 2.000 Monomereinheiten der Formeln (**IIIa**) und (**IIIb**) enthalten, 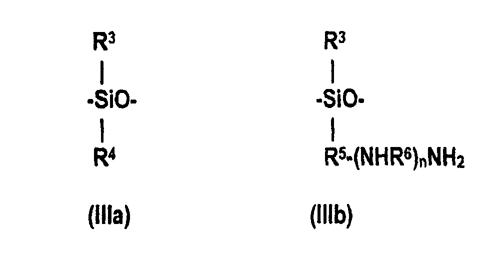 in der R³ und R⁴ unabhängig voneinander für Alkylgruppen mit 1 bis 8 Kohlenstoffatomen, R⁵ und R⁶ unabhängig voneinander für gegebenenfalls hydroxysubstituierte Alkylengruppen mit 1 bis 8 Kohlenstoffatomen und n für 0 oder Zahlen von 1 bis 3 steht.

7. Verfahren nach Anspruch 6, **dadurch gekennzeichnet, daß** man Aminosilicone der Formel (**III**) einsetzt, in der R³ und R⁴ für Methylgruppen stehen.

8. Verfahren nach den Ansprüchen 6 und 7, **dadurch gekennzeichnet, daß** man Aminosilicone der Formel (**III**) einsetzt, die einen Stickstoffgehalt von 0,1 bis 5 Gew.-% aufweisen.

9. Verfahren nach den Ansprüchen 1 bis 8, **dadurch gekennzeichnet, daß** man die Aminosilicone in Mengen von 0,0001 bis 0,2 Gew.-% - berechnet auf die Glucoprotamine - einsetzt.

10. Verwendung von Aminosiliconen als Entschäumer und Stabilisatoren in der Kondensation von N-substituierten Propylendiaminen mit 2-Aminoglutarderivaten.

## Claims

1. A process for the production of glucoprotamines by condensation of N-substituted propylenediamines with 2-aminoglutaric derivatives, **characterized in that** the reaction is carried out in the presence of aminosilicones.

2. A process as claimed in claim 1, **characterized in that** N-substituted propylenediamines corresponding to formula (**I**):
**R**^{**1**}**-NH-CH**_{**2**}**CH**_{**2**}**CH**_{**2**}**NH**_{**2**} (I)
in which R¹ is a linear alkyl group containing 6 to 22 carbon atoms, are used.

3. A process as claimed in claims 1 and 2, **characterized in that** 2-aminoglutaric derivatives corresponding to formula (**II**): 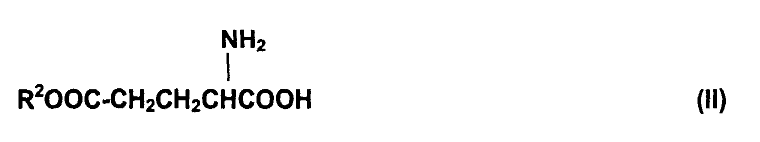 in which R² is hydrogen or a C₁₋₄ alkyl group, are used.

4. A process as claimed in claims 1 to 3, **characterized in that** the N-substituted propylenediamines and the 2-aminoglutaric derivatives are used in a molar ratio of 1:1 to 1:2.

5. A process as claimed in claims 1 to 4, **characterized in that** the condensation is carried out at temperatures of 60 to 175°C.

6. A process as claimed in claims 1 to 5, **characterized in that** aminosilicones containing 50 to 2,000 monomer units corresponding to formulae (**IIIa**) and (**IIIb**): 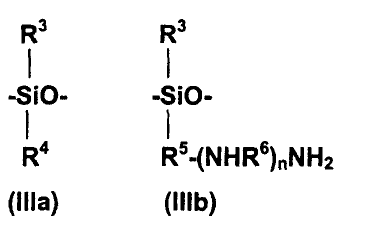 in which R³ and R⁴ independently of one another represent alkyl groups containing 1 to 8 carbon atoms, R⁵ and R⁶ independently of one another represent optionally hydroxysubstituted alkylene groups containing 1 to 8 carbon atoms and n is 0 or a number of 1 to 3, are used.

7. A process as claimed in claim 6, **characterized in that** aminosilicones corresponding to formula (III) in which R³ and R⁴ are methyl groups are used.

8. A process as claimed in claims 6 and 7, **characterized in that** aminosilicones corresponding to formula (III) with a nitrogen content of 0.1 to 5% by weight are used.

9. A process as claimed in claims 1 to 8, **characterized in that** the aminosilicones are used in quantities of 0.0001 to 0.2% by weight, based on the glucoprotamines.

10. The use of aminosilicones as antifoam agents and stabilizers in the condensation of N-substituted propylenediamines with 2-aminoglutaric derivatives.

## Revendications

1. Procédé de préparation de glucoprotamines par condensation de propylènediamines N- substituées avec des dérivés 2- aminoglutariques
**caractérisé en ce qu'**
on effectue la réaction en présence d'aminosilicones.

2. Procédé selon la revendication 1,
**caractérisé en ce qu'**
on met en oeuvre des propylènediamines N- substitués de formule (I),
R¹-NH-CH₂CH₂CH₂NH₂ (I)
dans laquelle R¹ représente un reste alkyle linéaire ayant de 6 à 22 atomes de carbone.

3. Procédé selon les revendications 1 et 2,
**caractérisé en ce qu'**
on met en oeuvre des dérivés d'acide 2- aminoglutarique de formule (II) 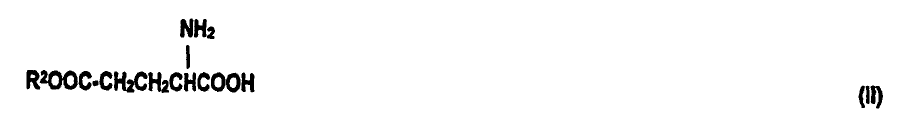 dans laquelle R² représente de l'hydrogène ou un reste alkyle ayant de 1 à 4 atomes de carbone.

4. Procédé selon les revendications 1 à 3,
**caractérisé en ce qu'**
on met en oeuvre les propylènediamines N- substitués et le dérivé d'acide 2- aminoglutarique dans un rapport molaire de 1 :1 à 1 :2.

5. Procédé selon les revendications 1 à 4,
caractérisé en qu'
on effectue la condensation à des températures dans la zone de 60 à 175°C.

6. Procédé selon les revendications 1 à 5,
**caractérisé en ce qu'**
on met en oeuvre des aminosilicones qui contiennent de 50 à 2000 éléments monomères de formules (IIIa) et (IIIb) 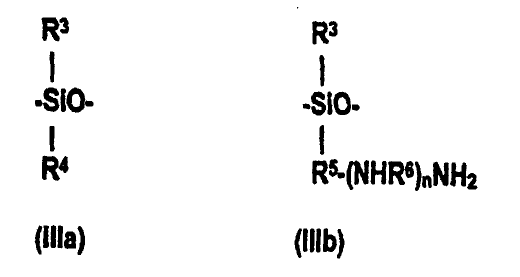 dans lesquelles R³ et R⁴ indépendamment l'une de l'autre, représentent des groupes alkyle ayant de 1 à 8 atomes de carbone, R⁵ et R⁶ indépendamment l'un de l'autre, représentent des groupes alkylène éventuellement substitués par un groupe hydroxy, ayant de 1 à 8 atomes de carbone, et n représente 0 ou des nombres allant de 1 à 3.

7. Procédé selon la revendication 6,
**caractérisé en ce qu'**
on met en oeuvre des aminosilicones de formule (III) dans laquelle R³ et R⁴ représentent des groupes méthyle.

8. Procédé selon les revendications 6 et 7,
**caractérisé en ce qu'**
on met en oeuvre des aminosilicones de formule (III) qui possèdent une teneur en azote de 0,1 à 5 % en poids.

9. Procédé selon les revendications 1 à 8,
**caractérisé en ce qu'**
on met en oeuvre des aminosilicones en quantités allant de 0,0001 à 0,2 % en poids calculé sur la glucoprotamine.

10. Utilisation des aminosilicones en tant qu'agents anti-mousse et comme agents stabilisants dans la condensation de propylènediamines N- substitués avec des dérivés 2- aminoglutariques.
